# EUROPEAN PATENT SPECIFICATION

(11) **EP 0 415 915 B1**
(45) Date of publication and mention of the grant of the patent: **09.10.1996**
(21) Application number: 88904139.8
(22) Date of filing: 20.04.1988
(51) Int. Cl.: A61B 19/00

(54) **APPARATUS FOR ANCHORING TISSUE**
GERÄT ZUR FIXIERUNG VON GEWEBE
APPAREIL POUR LA FIXATION DE TISSU

(43) Date of publication of application: 13.03.1991
(60) Divisional application: 95118658.4
(73) Proprietor: HAYHURST, John O., Milwaukie, OR 97267 (US)
(72) Inventor: HAYHURST, John O., Milwaukie, OR 97267 (US)
(74) Representative: Meddle, Alan Leonard
(86) International application number: PCT/US88/01286
(87) International publication number: WO 89/10096

(56) References cited:
- EP-A- 0 241 240
- WO-A-86/03666
- WO-A-87/01270
- GB-A- 2 118 474
- US-A- 3 470 834
- US-A- 3 990 619
- US-A- 4 235 238
- US-A- 4 275 717
- US-A- 4 326 531
- US-A- 4 462 395

## Description

### Technical Field

This invention relates to an apparatus for manipulating and anchoring cartilage and similar fibrous tissue within a joint.

### Background Art

Conventional medical clamps have certain disadvantages when used for manipulating cartilage or other tissue within a joint during arthroscopic surgery. Primarily, the clamps have a tendency to slip off the cartilage. Additionally, the size of the clamps in relation to the relatively small space within the joint makes it difficult to maneuver other surgical instruments, such as a scalpel or arthroscope, within the confined space of the joint. Such clamps can also interfere with the view of the inside of the joint afforded by the arthroscope. Since the clamps must be introduced into the joint through an incision, they are limited in their range of manipulation by the location of the incision. In order to apply a desired directional traction to the cartilage, it may be necessary to release the clamp from the cartilage, reintroduce the clamp through another incision, and reclamp the cartilage.

It is often necessary to repair torn fibrous tissue, such as a ligament or tendon, or reattach such tissue to bone. While in some instances it is possible to insert two needles into the joint and then thread both of them with a suture to form a loop to reattach torn parts of fibrous tissue, that procedure is undesirable because it is complex and time-consuming. The alternative of more radical arthrotomy is also undesirable because of the increased amount of trauma and resultant increased morbidity encountered in the use of such a procedure.

As is explained in the following, the present invention provides a relatively compact and easy to use apparatus for manipulating cartilage and other fibrous tissue, and for anchoring the tissue to other tissue or to bone. Some technical references that may be of general interest are as follows: Allen, U.S. Patent 3,699,969; Shein, U.S. Patent 3,527,223; Woo, U.S. Patent 3,943,932; Almen, U.S. Patent 3,500,820; Johnson et al., U.S. Patent 3,871,368; and Smith, U.S. Patent 4,243,037. None of these references discloses a method or apparatus suitable for manipulating fibrous tissue during arthroscopic surgery, or for effectively reattaching fibrous tissue to bone or to other fibrous tissue.

International publication No. WO 87/01270 discloses a resilient surgical fastener comprising a head which is relatively stiff along its length and a filament portion which is relatively flexible along its length, the two portions being arranged in a T configuration such that when the relatively stiff head portion is inserted into body tissue, the relatively flexible portion causes the relatively stiff portion to rotate and implant itself in the body tissue thus securing the surgical fastener to the tissue.

The aforementioned problems associated with use of conventional medical clamps for manipulating tissue are overcome by the present invention, which provides an apparatus according to claim 1 for manipulating and anchoring tissue during arthroscopic surgery. The apparatus provides adequate fixation of the tissue during such surgery and minimally interferes with the use of other instruments within the joint.

The apparatus particularly comprises an anchor member having a suture attached at its base. The anchor member is inserted through the tissue with the suture extending therefrom to provide a mechanism for manipulating the tissue within the joint.

The preferred means of inserting the anchor member includes a hollow needle having a sharp tip and an open butt. A hollow tube of equal or greater length than the needle slides within the needle. A limiting mechanism is provided at the butt of the needle and at the corresponding portion of the hollow tube to selectively position the tube within the needle so that the tube does not extend outwardly beyond the tip of the needle.

The anchor member is located within the tip of the hollow needle in a deformed U shape. The suture extends from the anchor member through the bore of the tube.

A removable shield fits over the tip of the needle to prevent the sharp tip from cutting the suture or the anchor member during the process of inserting the anchor member into the hollow needle.

It is often desirable to permanently reattach to bone fibrous tissue, such as tendons or ligaments. An alternative embodiment of a tissue anchoring apparatus is provided for that purpose. More particularly, the apparatus of this embodiment includes a deformable anchor member that has a base and at least two legs. Each leg is attached to the base and extends therefrom to terminate in an outer end. A suture is attached to the base of the anchor member. The anchor member is formed of resilient material for urging the anchor member into a relaxed position wherein the ends of the legs are spaced apart a maximum distance. The anchor member is deformable into a deformed position wherein the ends of the legs are spaced apart a minimum distance that is less than the maximum distance.

While in the deformed position, the anchor member is insertable into a hole that is drilled into the bone at the location the tissue is to be attached to the bone. The hole has a diameter that is less than the maximum distance between the ends of the anchor member legs. Consequently, upon insertion of the anchor member into the hole, the ends of the anchor member legs bear upon the bone within the hole, and the suture extends from the hole. Whenever tension is applied to the suture, the ends of the legs dig into the bone and resist removal of the anchor member from the hole.

With the anchor member anchored in the hole, the suture is available for securing the tissue to the bone. One way of using the suture to secure the tissue to the bone is to attach a retainer to the suture for pressing the tissue against the bone. The retainer includes resilient suture-engaging edges and corners, and is slidable along the suture in one direction, but grips the suture to resist sliding in the opposite direction. The retainer thereby holds tissue against the bone during healing so that the tissue will properly reattach to the bone.

To avoid prolonged irritation of surrounding tissues, the anchor member, suture, and retainer of the present invention may be made of material that is gradually absorbable by the body.

The foregoing and other features of the invention will be more readily understood upon consideration of the following detailed description of the invention, taken in conjunction with the accompanying drawings.

Fig. 1 is a perspective view of the inner surface and an edge of a retainer used in association with the suture and the anchor member for securing tissue to bone or to other tissue.

Fig. 2 is a perspective view of the outer surface and an edge of the retainer shown in Fig. 1.

Fig. 3 is a perpspective view of an alternative retainer.

Fig. 4 illustrates a portion of a joint in which the anchor member, suture and retainer are used to connect and retain a piece of cartilage in position against another piece of cartilage from which it had been torn.

Fig. 5 is a sectional view taken along line 14-14 of Fig. 4.

Fig. 6 is a side elevational view, partly in section, of an anchor member and suture that can be anchored to a bone.

Fig. 7 is a sectional elevational view of the anchor member and suture of Fig. 6, positioned within the preferred mechanism for inserting the anchor member into a hole in a bone.

Fig. 8 is a sectional elevational view showing the anchor member of Fig. 7 anchored within a hole in a bone and used, in conjunction with the suture and a retainer, to hold tissue against the bone.

Fig. 9 is a sectional elevational view showing an alternative method of using an anchor member and suture to hold tissue against the bone.

Fig. 10 is a side cross-sectional view of an alternative embodiment of an anchor member that has a hole formed therethrough to permit a suture to be looped through it.

Fig. 11 is a cross-sectional view of another alternative embodiment of an anchor member that can be anchored to a bone.

Fig. 12 is a top view of the anchor member of Fig.11.

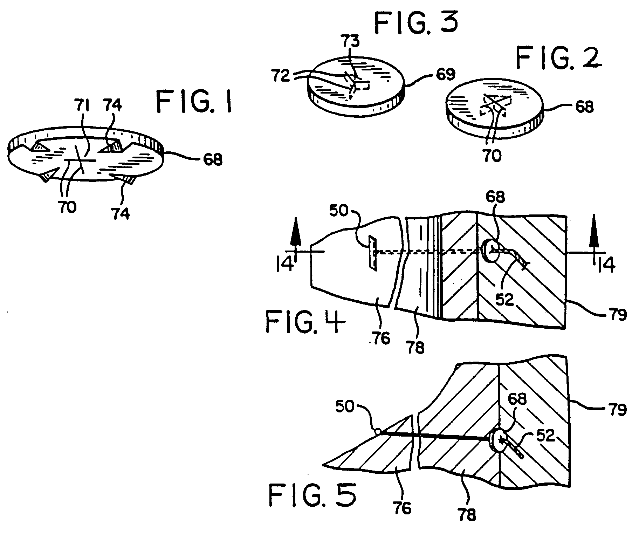

Referring now to Figs. 1-5, retainer devices 68 and 69, each having a pair of generally parallel surfaces, are made of resilient material and have slits 70 and 72, respectively, which intersect near the central points of the parallel surfaces, defining pointed corner flaps 71 and 73, respectively. The retainers 68 and 69 are preferably circular because the circular shape may reduce the possibility of irritation of surrounding tissue. It will be understood, however, that this shape is a matter of choice and that other shapes would also be acceptable.

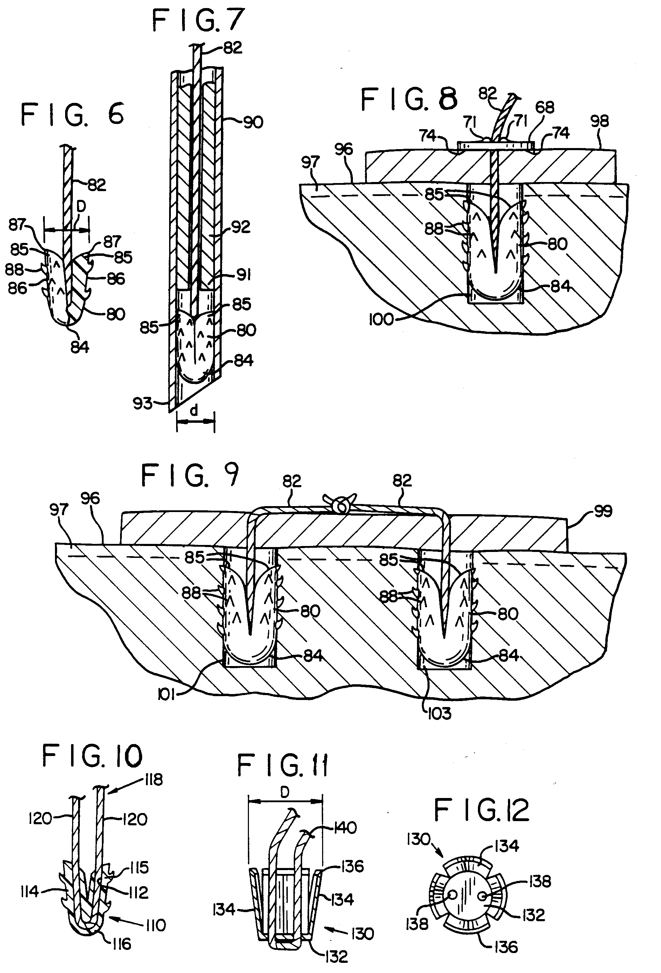

Raised points 74 are provided on the inner surface of the retainer 68 to bear against tissue, and to assist in immobilizing the tissue while the anchor member is in use. In many instances, however, the raised points 74 will not be required and a flat inner surface will suffice. The following discussion of retainer use is directed to the anchor member 50 of Figs 4 & 5 however, it is understood that the discussion applies to all embodiments of the anchor member described herein.

The retainer 68 (or retainer 69) may be used in conjunction with the anchor member 50 by inserting the free end 57 of the suture through the retainer at the intersection of the slits 70 after the hollow needle and hollow tube have been withdrawn from around the suture. When the suture 52 is inserted through the retainer 68, the flaps 71 that are defined between adjacent slits 70 are resiliently deformed toward the direction of movement of the suture therethrough. Thereafter, the flaps wedge against the suture 52 and resist withdrawal of the suture through the slits. By applying tension to the suture 52 (see Figs. 4 and 5) and urging the retainer 68 along the suture to the surface of cartilage 76 from which the suture extends, the retainer may be used to maintain tension in the suture, thereby holding a loose piece of cartilage 76 against the stable piece of cartilage 78 from which the loose piece of cartilage 76 had been torn or fractured.

The anchor member 50, suture 52, and retainer 68 may be left permanently in the joint to retain the torn cartilage 76 in its proper location against the stable cartilage 78, with the retainer 68 resting against the outside of the stable cartilage 78, between the surface of the stable cartilage 78 and muscle tissue 79 adjacent thereto.

It is noteworthy that in many instances the needle 54 may be inserted into a joint from opposing directions. For example, the anchor member 50 was deposited in the position shown in Figs. 4 and 5 by a needle that penetrated the muscle tissue 79. The needle could have been inserted from the opposing side of the joint (and not through muscle tissue 79) to deposit the anchor member 50 in the position occupied by the retainer 68 in Figs. 4 and 5. Accordingly, the positions of the anchor member 50 and the retainer 68 would be reversed from those shown in Figs.4 and 5, but the loose cartilage 76 would still be held against the secure cartilage 78. One reason for inserting the needle from the opposing side of the joint, as just explained, would be to avoid damaging any nerves or blood vessels that are present in the region of the muscle tissue 79.

To prevent prolonged irritation of the surrounding tissue by the presence of the anchor member 50 and retainer 68, it is particularly desirable to form the anchor member and retainer of material that can be gradually absorbed by the body of the patient as healing occurs. Resilient, synthetic materials that are gradually absorbable by the body are known for use in sutures and are desirable as materials for the anchor member and retainers of the present invention. One such material is an absorbable polymer known as poly-diaxanone (PDS), which is available from Ethicon, Inc., of Summerville, New Jersey.

Referring now to Figs. 6-8 an anchor member 80 is particularly adapted for use in anchoring a sure 82 to bone 96 so that the suture 82 may be used to reattach tissue 98 to the bone. The anchor member 80 is generally bullet-shaped having a rounded convex base 84 with two attached legs 86 extending from the base. The outer ends 85 of the legs are tapered and terminate in a sharp outer edges 87. The anchor member 80 is formed of resilient material, and whenever the anchor member is in its relaxed state (Fig. 6 ), the legs 86 diverge outwardly so that the outer edges 87 of the legs are spaced apart a maximum distance D. One end of a suture 82 is embedded within, or otherwise attached to, the base 84 of the anchor member 80. Suture 82 extends outwardly from the base 84 between the legs 86.

Preferably, the outer surface of the anchor member 80 carries a plurality of barbs 88. The barbs 88 point outwardly, and away from the rounded convex base 84. As a result, the exposed sharp point of each barb 88 is directed generally toward the direction in which the suture 82 extends away from the base 84 of the anchor member 80.

As shown in Fig. 7 the anchor member 80 is inserted within the tip 93 of a hollow needle 90 ahead of the tip 91 of a tube 92 that is used to expel the anchor member 80 from the needle. The suture 82 extends through the bore of the tube 92.

The anchor member 80 and the bore of the needle 90 are sized so that the anchor member is in a deformed position whenever it is lodged within the tip 93 of the needle. In the deformed position, the legs 86 of the anchor member are pressed together with the outer edges 87 of the legs being spaced apart a minimum distance d corresponding to the needle bore diameter. This distance d is less than the maximum. distance D between the outer edges 87 as measured when the anchor member is in the relaxed position (Fig.6).

As noted, the anchor member 80 is formed of resilient material. Consequently, whenever the anchor member 80 is expelled from the needle 90, the intrinsic resilience of the anchor member urges it into the relaxed position. As will now be explained, the tendency of the anchor member 80 to move from the deformed into the relaxed position provides a simple mechanism for anchoring the anchor member 80 in bone so that, in conjunction with the attached suture, there is provided a means for reattaching tissue to the bone to promote healing.

More particularly, with reference to Fig. 8, a hole 100 is drilled into the bone 96 in the region where the tissue 98 is to be reattached to the bone. The hole diameter is less than the maximum distance D between the outer edges 87 of the anchor member, but greater than or equal to the bore diameter of the needle 90. With the anchor member 80 within the tip 93 of the needle 90, the tissue 98 is pierced by the needle in a manner as described earlier. The tip 93 of the needle is forced through the tissue 98 and then aligned with the hole 100. Next, the anchor member 80 is expelled from the needle into the hole 100 by sliding the tube 92 toward the tip 93 of the needle 90.

Once expelled from the needle 90 into, the hole 100, the resilience of the anchor member 80 urges the outer edges 87 of the legs 86 to bear upon the bone within the hole 100. With the outer edges 87 of the legs bearing upon the bone, any tension applied to the suture 82 causes the sharp edges 87 to dig into the bone to secure the anchor member within the hole. The barbs 88 also dig into the bone to supplement the anchoring effect of the legs 86.

Preferably, the anchor member 80 is sized so that when it is positioned within the hole 100, the outer edges 87 of the legs 86 are beneath a relatively dense bone layer 97 that is located at the surface of the bone 96, and is known as the cortical layer 97. As a result, tension in the suture (in conjunction with the intrinsic resilient force of the anchor member 80 that forces the leg edges 87 apart) tends to lodge the edges 87 of the anchor member legs beneath the cortical layer 97, rendering the anchor member substantially irremovable from the hole 100.

As shown in Fig.8, a retainer 68, as described earlier, nay be employed with the suture 82 to secure the tissue 98 to the bone 96.

Fig. 9 illustrates another technique for securing tissue 99 to the bone 96, wherein two anchor members 80 are anchored in holes 101, 103, and the free ends of the sutures 82 are tied together over the tissue.

It is noted that it may not be necessary to first pierce the tissue 99 before depositing the anchor member 80 into the hole 101, 103. For instance, the anchor member 80 may be deposited within the hole 101, 103 in the manner described above, and the free end of the suture 82 may be threaded through a conventional surgical needle that is used to pierce the tissue. The surgical needle is then removed and the free ends of the sutures 82 are secured as described above.

Fig.10 depicts an alternative embodiment of an anchor member 110 suitable for anchoring in bone. The anchor member 110 is substantially similar to the anchor member 80 described earlier, except that it includes a continuous passage 112 formed therein to pass into one leg 114, through the base 116, and out the other leg 115. The suture 118 is threaded through the hole passage 112 so that two suture segments 120 extend from the anchor member. This configuration of the anchor member 110 allows the user to select any type of suture for use with the anchor member 110, depending upon the particular surgical needs. Further, having two suture segments 120 available for securing the tissue to the bone is often desirable. For example, whenever an odd number of anchor members 116 is used, the resulting even number of available suture segments 120 permits each segment of one anchor member to be tied to a corresponding segment of an adjacent anchor member, without the need for tying more than two suture segments together.

Figs. 11 and 12 illustrate a side sectional view and top view, respectively, of another alternative embodiment of an anchor member 130 formed in accordance with this invention. This embodiment is a generally cup-shaped piece of resilient material, such as plastic, having a base 132 with four legs 134 extending upwardly therefrom. The sharp outer edge 136 of each leg is spaced apart from an opposing edge 136 by a maximum distance D whenever the anchor member is in the relaxed position as shown in Fig.11 As noted earlier, distance D is greater than the diameter of the hole into which the anchor member 130 is deposited. Preferably, two holes 138 are formed in the base 132 of the anchor member 130. A suture 140 is threaded through the holes 138.

The anchor member 130 is deposited within a hole in a bone in a manner similar to that explained with respect to the apparatus of Fig. 7. Specifically, the anchor member 130 is positioned within the tip of a hollow needle (not shown) where it assumes a deformed position. In the deformed position, the outer edge 136 of each leg is held near the outer edge 136 of the opposing leg a distance d that is less than the "relaxed" distance D and corresponds to the diameter of the needle bore in which the anchor member is lodged. When the anchor member 130 is expelled from the needle and deposited within the hole in the bone, the intrinsic resilience of the anchor member 130 forces the outer edges 136 against the bone, thereby anchoring the anchor member within the hole. The suture 140 is thereafter available to secure tissue against the bone as discussed above.

The anchor members 80, 110, 130 just described may be formed of material that is absorbable by the body. Alternatively, the anchor members may be formed of non-absorbable material (e.g., stainless steel of suitible resilience) that remains in the bone indefinitely.

## Claims

1. An apparatus for insertion into bone for anchoring tissue to bone comprising, an anchor member (80) having a base (84) and two legs (86) attached to the base, the two legs being closely spaced such that the base defines an end of the anchor member whereby the anchor member is movable base-first into a hole (100) in a bone (96) with the ends (85) of the legs (86) that are not attached to the base following the base into the bone, the anchor member (80) being made of resilient material and being deformable so that the legs (86) are urged to diverge outwardly to lodge the anchor member (80) in the hole in the bone, and a suture (82) attached to the anchor member to extend from the anchor member to anchor the suture (82) in the bone (96) with the suture (82) extending from the bone (96).

2. The apparatus of Claim 1 further characterised in that the anchor member (110) has formed in it at least one aperture (112) through which the suture (120) is removably threaded to provide two suture segments extending from the anchor member and the bone.

3. The apparatus of Claim 1 or 2 further characterised in that the ends (85) of the legs are formed with sharp edges (87) that bear upon the bone when the anchor member is positioned within a hole in the bone, the edges being shaped to dig into the bone whenever tension is applied to the suture.

4. The apparatus of any preceding claim, wherein the anchor member is made of resilient material so that the closely spaced legs may be moved toward each other to facilitate placement of the anchor member within a hole (101) in the bone.

5. The apparatus of any preceding claim further characterised in that the legs (86) have outer surfaces having barbs (88) formed therein that contact the bone to resist removal of the anchor member (80) from the bone whenever tension is applied to the suture (82).

6. The apparatus of any preceding claim in combination with insertion means for inserting the anchor member (80) into the bone, the insertion means including:
a hollow needle having a tip (93) and a butt, the needle tip having a bore in which is completely received the anchor member (80) so that the suture (82) does not protrude from the needle tip; and
an expulsion member (92) positionable within the hollow needle to be slidable therein and operatively associated with the needle for expelling the anchor member (80) from the needle tip.

7. The apparatus of Claim 6, wherein the resilience of the anchor member (80) enables the closely spaced legs (86) to be moved towards each other to facilitate placement of the anchor member (80) within the bore of the needle tip (93).

8. The apparatus of any preceding claim further characterised in that portions of the legs (86) are spaced apart a maximum distance that is wider than the hole in the bone within which the anchor member (80) is to be inserted, the anchor member (80) being deformable into a position such that the legs (86) are spaced apart a minimum distance and, upon insertion of the anchor member (80) into the hole, the anchor member fits tightly in the hole.

## Patentansprüche

1. Vorrichtung zum Einsetzen in Knochen zur Fixierung von Gewebe, umfassend ein Verankerungsteil (80) mit einem Sockel (84) und zwei an dem Sockel gehaltenen Schenkein (86) wobei die beiden Schenkel einen geringen Abstand voneinander aufweisen, so daß der Sockel einen Endabschnitt des Verankerungsteils bildet, wodurch das Verankerungsteil mit dem Sockel voran in eine Bohrung (100) in einem Knochen (96) bewegbar ist, wobei die Enden (85) der Schenkel (86), die nicht am Sockel befestigt sind, dem Sockel in den Knochen hinein folgen, wobei das Verankerungsteil (80) aus elastischen Material besteht und verformbar ist, so daß die Schenkel (86) nach außen auseinandergedrückt werden, um das Verankerungsteil (80) in die Bohrung im Knochen fest einzusetzen; und einen Wundfaden (82), der an dem Verankerungsteil befestigt ist und sich von diesen erstreckt, so daß der Wundfaden (82) in dem Knochen (96) verankert wird und sich von dem Knochen (96) weg erstreckt.

2. Vorrichtung nach Anspruch 1, weiter dadurch gekennzeichnet, daß in dem Verankerungsteil (110) wenigstens eine Öffnung (112) ausgebildet ist, durch die der Wundfaden (120) lösbar eingefädelt ist, um zwei Wundfadenabschnitte bereitzustellen, die sich von dem Verankerungsteil und dem Knochen erstrecken.

3. Vorrichtung nach Anspruch 1 oder 2, weiter dadurch gekennzeichnet, daß die Endabschnitte (85) der Schenkel mit scharfen Kanten (87) ausgebildet sind, die auf dem Knochen anliegen, wenn das Verankerungsteil in eine Bohrung in dem Knochen eingesetzt wird, wobei die Kanten so geformt sind, daß sie in den Knochen eintreten, sobald ein Zug auf den Wundfaden ausgeübt wird.

4. Vorrichtung nach einem der vorangehenden Ansprüche, dadurch gekennzeichnet, daß das Verankerungsteil aus elastischem Material besteht, so daß die eng benachbarten Schenkel zueinander bewegt werden können, um das Einsetzen des Verankerungsteils in eine Bohrung (101) in dem Knochen zu erleichtern.

5. Vorrichtung nach einem der vorangehenden Ansprüche, weiter dadurch gekennzeichnet, daß die Schenkel (86) Außenflächen aufweisen, in denen Widerhaken (88) ausgebildet sind, die mit dem Knochen in Berührung stehen, um einem Herausziehen des Verankerungsteils (80) aus dem Knochen zu widerstehen, sobald ein Zug auf den Wundfaden (82) ausgeübt wird.

6. Vorrichtung nach einem der vorangehenden Ansprüche, in Kombination mit einer Einsetzeinrichtung zum Einsetzen des Verankerungsteils (80) in den Knochen, wobei die Einsetzeinrichtung umfaßt:
eine hohle Nadel mit einer Spitze (93) und einem stumpfen Ende, wobei die Spitze der Nadel eine Bohrung aufweist, in der das Verankerungsteil (80) vollständig aufgenommen ist, so daß der Wundfaden (82) aus der Spitze der Nadel nicht hervorsteht; und
ein Ausstoßteil (92), das innerhalb der hohlen Nadel positioniert werden kann, um darin verschiebbar zu sein, und das der Nadel wirkungsmäßig zugeordnet ist, um das Verankerungsteil (80) aus der Nadelspitze auszustoßen.

7. Vorrichtung nach Anspruch 6, dadurch gekennzeichnet, daß die Elastizität des Verankerungsteils (80) ermöglicht, daß sich die eng benachbarten Schenkel (86) zueinanderbewegen, um das Einsetzen des Verankerungsteils (80) in die Bohrung der Nadelspitze (93) zu erleichtern.

8. Vorrichtung nach einem der vorangehenden Ansprüche, weiter dadurch gekennzeichnet, daß Abschnitte der Schenkel (86) einen maximalen Abstand voneinander aufweisen, der größer ist als die Bohrung in dem Knochen, in die das Verankerungsteil (80) einzusetzen ist, wobei das Verankerungsteil (80) in eine Stellung verformbar ist, in der die Schenkel (86) einen minimalen Abstand voneinander aufweisen und das Verankerungsteil beim Einsetzen in die Bohrung eng in der Bohrung sitzt.

## Revendications

1. Un appareil d'insertion dans l'os pour ancrer du tissu comprenant,
un élément d'ancrage (80) ayant une base (84) et deux pattes (86) liées à la base, les deux pattes étant espacées étroitement de sorte que la base définisse une extrémité de l'élément d'ancrage afin que l'élément d'ancrage soit mobile d'abord dans un trou (100) dans un os (96) avec les extrémités (85) des pattes (86) qui ne sont pas liées à la base à la suite de la base dans l'os, l'élément d'ancrage (80) étant en matière élastique et étant déformable afin que les pattes (86) soient poussées pour diverger vers l'extérieur de manière à loger l'élément d'ancrage (80) dans le trou de l'os, et
un fil de suture (82) lié à l'élément d'ancrage à étendre depuis l'élément d'ancrage pour ancrer le fil de suture (82) dans l'os (96) avec le fil de suture (82) s'étendant depuis l'os (96).

2. L'appareil de la revendication 1 caractérisé en outre en ce que dans l'élément d'ancrage (110) est ménagé au moins un orifice (112) à travers lequel le fil de suture (120) est enfilé de manière amovible pour fournir deux segments de fil de suture s'étendant depuis l'élément d'ancrage et l'os.

3. L'appareil de la revendication 1 ou 2 caractérisé en outre en ce que les extrémités (85) des pattes sont formées avec des bords tranchants (87) qui portent sur l'os lorsque l'élément d'ancrage est positionné dans un trou de l'os, les bords étant formés pour s'enfoncer dans l'os chaque fois qu'une tension est appliquée au fil de suture.

4. L'appareil d'une quelconque revendication précédente, dans lequel l'élément d'ancrage est en matière élastique de sorte que les pattes espacées étroitement puissent être déplacées l'une vers l'autre pour faciliter le placement de l'élément d'ancrage dans un trou (101) de l'os.

5. L'appareil d'une quelconque revendication précédente caractérisé en outre en ce que les pattes (86) ont des surfaces externes ayant des barbes externes (88) formées dans celles-ci qui viennent au contact de l'os pour résister au retrait de l'élément d'ancrage (80) de l'os chaque fois qu'une tension est appliquée au fil de suture (82).

6. L'appareil d'une quelconque revendication précédente en combinaison avec un moyen d'insertion pour insérer l'élément d'ancrage (80) dans l'os, le moyen d'ancrage incluant :
une aiguille creuse ayant une pointe (93) et une butée, la pointe d'aiguille ayant un alésage dans lequel est complètement reçu l'élément d'ancrage (80) de sorte que le fil de suture (82) ne saille pas de la pointe d'aiguille ; et
un élément d'expulsion (92) positionnable dans l'aiguille creuse pouvant coulisser dans celle-ci et associé fonctionnellement à l'aiguille pour expulser l'élément d'ancrage (80) de la pointe d'aiguille.

7. L'appareil de la revendication 6, dans lequel l'élasticité de l'élément d'ancrage (80) permet aux pattes espacées étroitement (86) d'être déplacées l'une vers l'autre pour faciliter le placement de l'élément d'ancrage (80) dans l'alésage de la pointe d'aiguille (93).

8. L'appareil d'une quelconque revendication précédente caractérisé en outre en ce que des portions des pattes (86) sont espacées d'une distance maximale qui est plus grande que le trou dans l'os à l'intérieur duquel l'élément d'ancrage (80) est à insérer, l'élément d'ancrage (80) étant déformable à une position telle que les pattes (86) soient espacées d'une distance minimale et, lors de l'insertion de l'élément d'ancrage (80) dans le trou, l'élément d'ancrage soit serré fermement dans le trou.
